# FASCICULE DE BREVET EUROPEEN

(11) **EP 3 435 924 B1**
(45) Date de publication et mention de la délivrance du brevet: **27.05.2020**
(21) Numéro de dépôt: 17713789.0
(22) Date de dépôt: 27.03.2017
(51) Int. Cl.: A61F 2/30, A61B 17/70, A61F 2/44, A61F 2/46, A61B 90/00

(54) **PROCÉDÉ DE FABRICATION D'UN IMPLANT, NOTAMMENT VERTÉBRAL OU INTERVERTÉBRAL, ET IMPLANT OBTENU PAR CE PROCÉDÉ**
VERFAHREN ZUR HERSTELLUNG EINES IMPLANTATS, INSBESONDERE EINES WIRBEL- ODER ZWISCHENWIRBELIMPLANTATS, UND DURCH DIESES VERFAHREN HERGESTELLTES IMPLANTAT
METHOD FOR MAKING AN IMPLANT, NOTABLY A VERTEBRAL OR INTERVERTEBRAL IMPLANT, AND IMPLANT OBTAINED BY THIS METHOD

(30) Priorité: 30.03.2016 FR 1652714
(43) Date de publication de la demande: 06.02.2019
(73) Titulaire: Medicrea International, 69140 Rillieux La Pape (FR)
(72) Inventeur: SOURNAC, Denys, 01600 Reyrieux (FR); MOSNIER, Thomas, 69270 Rochetaillée sur Saône (FR); RYAN, David, 69660 Collonges au Mont d'Or (FR)
(74) Mandataire: LLR
(86) Numéro de dépôt international: PCT/IB2017/051733
(87) Numéro de publication internationale: WO 2017/168304

(56) Documents cités:
- WO-A2-2005/037137
- DE-A1-102005 004 563
- DE-A1-102005 033 608
- DE-U1-202015 001 700
- FR-A1- 3 001 381
- US-A1- 2003 039 676

## Description

La présente invention concerne un procédé de fabrication d'un implant, notamment vertébral ou intervertébral, et l'implant obtenu par ce procédé.

Il est bien connu d'immobiliser deux vertèbres l'une par rapport à l'autre au moyen d'un implant intervertébral en matériau rigide, formant une cage qui délimite un logement, ce logement étant destiné à recevoir un ou plusieurs greffons osseux et/ou des copeaux d'os spongieux. Certaines cages intervertébrales sont implantées par une voie d'abord postérieure et sont dites "PLIF" (acronyme de l'anglais *Posterior Lumbar Interbody Fusion*), d'autres par une voie d'abord antérieure ("ALIF", *Anterior Lumbar Interbody Fusion*), et d'autres encore par une voie d'abord transforaminale ("TLIF", *Transforaminal Lumbar Interbody Fusion*).

Pour assurer l'ancrage de cages PLIF ou ALIF, il est connu de prévoir des éléments déployables depuis l'intérieur de la cage, sous forme de griffes ou de pointes d'ancrage ; il est aussi connu de prévoir une cage ayant une partie antérieure (c'est-à-dire la partie de la cage située du côté antérieur de la vertèbre après implantation) dont la hauteur est apte à être augmentée après implantation, de façon à permettre, au moyen de cette cage, de redonner à des vertèbres lombaires leur écartement mutuel et leur lordose anatomique. Une telle cage comprend une structure de base à deux branches et une pièce formant un coin, mobile entre ces branches, cette pièce formant coin permettant, par son déplacement par rapport auxdites branches après implantation de la cage, de réaliser le déploiement de la partie antérieure de la cage.

Les cages existantes sont en métal, en général en titane ou en acier inoxydable ; elles ont une structure relativement complexe, et leur fabrication est également complexe et onéreuse.

Une cage TLIF, telle que décrite par exemple dans la publication de demande de brevet N° EP 2 459 126 au nom de la demanderesse, doit quant à elle être reliée à un instrument permettant son insertion dans un espace intervertébral, en étant maintenue dans le prolongement de cet instrument lors de son insertion dans ledit espace intervertébral, puis, une fois insérée dans cet espace, en étant apte à être pivotée par rapport à l'instrument pour prendre sa place dans la zone antérieure de l'espace intervertébral, et doit enfin être séparée de l'instrument.

La zone de liaison d'une telle cage avec l'instrument est en général complexe et relativement onéreuse à fabriquer. De plus, les cages et instruments existants n'excluent pas tout risque de positionnement défectueux de la cage.

Il est également bien connu de mettre en place sur des vertèbres, des organes d'ancrage à des vertèbres, tels que notamment des vis pédiculaires ou des crochets lamaires, permettant le montage sur ces vertèbres de barres rigides d'immobilisation des vertèbres. Un tel organe d'ancrage est fréquemment "polyaxial", c'est-à-dire qu'il comprend une partie distale d'ancrage osseux et une partie proximale de liaison à une barre, articulée par rapport à cette partie de base. Ladite partie proximale de liaison peut être sous la forme d'une pièce de réception d'une barre (une telle partie proximale est dite "tulipe" en référence à sa forme extérieure), ou peut être sous la forme d'un pion articulé, tel que décrit notamment par la publication de demande de brevet N° WO 98/55038, aussi au nom de la demanderesse.

De tels organes d'ancrage sont également relativement complexes et onéreux à fabriquer.

Les publications de demande de brevet N° DE 10 2005 033608 A1 et N° DE 10 2005 004563 A1 décrivent des procédés de fabrication d'un implant comprenant au moins deux pièces, dont au moins une première pièce est destinée à être mobile par rapport à au moins une deuxième pièce, ces procédés comprenant l'étape consistant à concevoir l'implant de telle sorte que ladite deuxième pièce enveloppe au moins partiellement ladite première pièce. La partie pré-caractérisante de la revendication 1 annexée est basée sur cet art antérieur.

Les implants décrits par publications de demandes de brevet ou de modèle d'utilité N° DE 20 2015 001700 U1, WO 2005/037137 A2, US 2003/039676 A1 et FR 3 001 381 A1 ne permettent pas de solutionner les inconvénients précités.

La présente invention a pour but de remédier à l'ensemble des inconvénients précités.

Selon l'invention, le procédé comprend en outre les étapes suivantes :
- concevoir l'implant de telle sorte qu'au moins un pont de liaison relie ladite première pièce à ladite deuxième pièce, chaque pont présentant une section telle qu'il est ruptible ;
- réaliser l'implant par un procédé dit d"'impression 3D", consistant :
   - à déposer sur un plateau une couche de poudre de matériau apte à être fusionné,
   - à opérer une fusion des particules de cette couche de poudre en des emplacements correspondant à la forme qu'ont, au niveau de cette couche, ladite première pièce et/ou ladite deuxième pièce et/ou ledit au moins un pont, selon que cette première pièce, cette deuxième pièce et/ou ce pont sont présents ou non au niveau de la section qu'a l'implant à constituer au niveau de cette couche ;
   - à reproduire ces opérations jusqu'à la constitution complète de l'implant, et
   - à éliminer les particules de poudre non fusionnées.

Ladite première pièce de l'implant est ainsi réalisée en même temps que ladite deuxième pièce de l'implant, avec ladite deuxième pièce enveloppant au moins partiellement ladite première pièce, de même qu'au moins un pont ruptible reliant ladite première pièce à ladite deuxième pièce, et l'enveloppement au moins partiel de ladite première pièce par ladite deuxième pièce réalise un assemblage de cette première pièce à cette deuxième pièce lorsque chaque pont est rompu.

Le procédé selon l'invention permet ainsi de réaliser un implant de façon relativement simple et rapide, sans opérations d'assemblage ultérieures.

La ruptibilité de chaque pont peut être obtenue en donnant à chaque pont une section réduite, par exemple inférieure à 1 mm².

Cette ruptibilité peut être réalisée en usine, de façon que d'éventuels débris qu'elle pourrait générer soient éliminés au stade de la fabrication. Il est cependant concevable que la rupture de chaque pont se fasse au moment de l'intervention chirurgicale, par une action manuelle ou au moyen d'un instrument.

L'invention concerne également l'implant obtenu directement par ce procédé.

L'implant peut notamment être une cage intervertébrale ayant une partie antérieure dont la hauteur est apte à être augmentée après implantation ; ladite deuxième pièce est alors constituée par un corps de cage formant deux branches, qui est déformable de cette sorte que des portions d'extrémité antérieures de ces branches soient mobiles l'une par rapport à l'autre entre une position de rapprochement mutuel et une position d'écartement mutuel ; ladite première pièce est alors constituée par un coin d'écartement engagé entre lesdites branches, relié au corps de cage par ledit au moins un pont.

L'implant peut également être une cage intervertébrale de type "TLIF" ; ladite première pièce peut alors être constituée par une pièce de révolution et ladite deuxième pièce être constituée par un corps de cage formant un palier de réception et rétention de cette pièce de révolution ; la pièce de révolution comprend un moyen de liaison à un instrument d'introduction / positionnement de l'implant.

Ladite pièce de révolution et ledit palier permettent la mobilité en pivotement de ladite deuxième pièce par rapport à ladite première pièce, requise par ce type de cage intervertébrale.

Ladite pièce de révolution peut notamment être un cylindre et son moyen de liaison audit instrument peut notamment être formé par un trou traversant formant un filet interne aménagé dans cette pièce de révolution. Un instrument de mise en place de la cage comprend alors une tige présentant un embout distal fileté destiné à être vissé dans ce trou traversant de ladite pièce de révolution, la venue de cet embout distal fileté contre la paroi de ladite deuxième pièce délimitant le palier permettant d'immobiliser ladite deuxième pièce dans une position déterminée par rapport à l'instrument, notamment dans une position dans laquelle cette deuxième pièce est dans le prolongement longitudinal de l'instrument.

L'implant peut également être une vis pédiculaire polyaxiale ou un crochet lamaire polyaxial ; ladite première pièce est alors formée par une tête de connexion ou un pion fileté, et ladite deuxième pièce est destinée à venir en engagement avec une vertèbre à traiter.

Selon une forme de réalisation préférée de l'invention, dans ce cas, l'une parmi ladite première pièce et ladite deuxième pièce présente une portion de forme au moins partiellement sphérique et l'autre parmi cette première pièce et cette deuxième pièce présente une cavité de forme au moins partiellement sphérique correspondante, destinée à recevoir cette portion de forme au moins partiellement sphérique, ledit pont de matière étant aménagé entre cette portion de forme au moins partiellement sphérique et la paroi délimitant cette cavité de forme au moins partiellement sphérique.

L'invention sera bien comprise, et d'autres caractéristiques et avantages de celle-ci apparaîtront, en référence au dessin schématique annexé, représentant, à titre d'exemples non limitatifs, plusieurs formes de réalisation de l'implant qu'elle concerne.
La figure 1 est une vue en perspective de l'implant selon une première forme de réalisation, constitué dans ce cas par une cage intervertébrale de type ALIF, de largeur adaptée à une mise en place latérale dans un espace intervertébral ;
la figure 2 en est une vue en bout, du côté proximal ;
la figure 3 en est une vue schématique, en coupe longitudinale ;
la figure 4 est une vue en perspective de l'implant selon une deuxième forme de réalisation, constitué également par une cage intervertébrale de type ALIF, ayant une largeur adaptée à une mise en place centrale dans un espace intervertébral ;
la figure 5 est une vue de côté de l'implant selon une troisième forme de réalisation, constitué dans ce cas par une cage intervertébrale de type TLIF ;
les figures 6 à 8 sont des vues de cet implant en coupe selon respectivement les lignes VI-VI de la figure 5 et VII-VII et VIII-VIII de la figure 6 ;
les figures 9 à 11 sont des vues de dessus de cet implant et de l'instrument de mise en place de celui-ci, au cours de différentes étapes successives de la mise en place de l'implant dans un espace intervertébral ;
la figure 12 est une vue en perspective de l'implant selon une quatrième forme de réalisation, constitué dans ce cas par une vis pédiculaire à pion proximal articulé ;
la figure 13 est une vue de l'implant en coupe longitudinale passant par l'axe de la vis, ledit pion proximal étant immobilisé dans une position axiale ;
la figure 14 est une vue de l'implant similaire à la figure 13, ledit pion proximal étant dans une position inclinée ; et
les figures 15 et 16 sont des vues de l'implant selon une cinquième forme de réalisation, homologues aux figures 12 et 13 respectivement, dans le cas d'un crochet lamaire à pion proximal articulé.

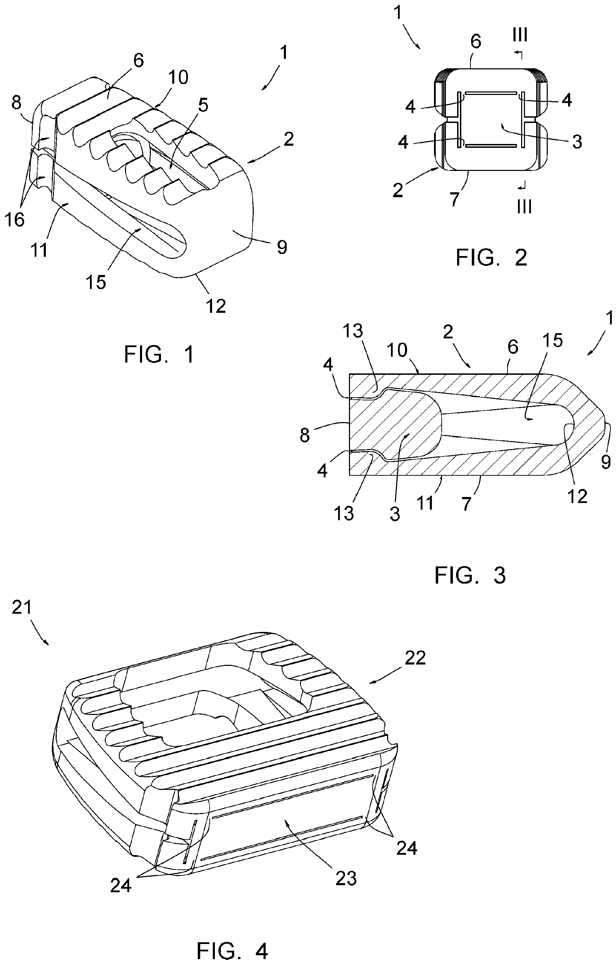

Les figures 1 à 3 représentent une cage intervertébrale 1 de type ALIF, c'est-à-dire destinée à être implantée par une voie d'abord antérieure.

La cage 1 est constituée par un corps de cage 2, par un coin d'écartement 3 et par quatre ponts de matière 4 reliant ce coin 3 et le corps de cage 2, comme visible sur les figures 2 et 3.

L'ensemble de la cage 1 est réalisé par un procédé dit d"'impression 3D", qui consiste :
- à déposer sur un plateau une couche de poudre de matériau apte à être fusionné,
- à réaliser une fusion des particules de cette couche de poudre en des emplacements déterminés, correspondant à une forme qu'a une section de la cage 1 à réaliser au niveau de cette couche,
- à reproduire ces opérations jusqu'à la constitution de la cage 1 complète, et
- à éliminer les particules de poudre non fusionnées.

Lesdits emplacements déterminés sont donc délimités, d'une couche à l'autre, par les contours qu'ont, au niveau de la couche considérée, le corps de cage 2, le coin d'écartement 3 et lesdits ponts 4, pour autant que ce corps de cage 2, ce coin d'écartement 3 et ces ponts 4 soient présents au niveau de la section qu'a la cage 1 à constituer au niveau de cette couche.

Le coin 3 est donc réalisé par ce procédé en même temps que le corps de cage 2, de même que les ponts 4 de matière reliant le coin 3 au corps de cage 2.

Le corps de cage 2 présente une cavité 5 débouchant sur l'extérieur de ce corps par de larges ouvertures, cette cavité 5 étant destinée à être remplie d'un ou plusieurs greffons d'os spongieux ou d'une agglomération de copeaux d'os spongieux.

Ce corps de cage 2 présente une forme générale parallélépipédique, définissant une face supérieure 6 dentelée, une face inférieure 7 opposée à cette face supérieure 6, une extrémité proximale 8 et une extrémité distale 9.

Il sera compris que les termes "supérieure" et "inférieure" se réfèrent à la position que la cage 1 est destinée à avoir lorsqu'elle est implantée entre deux vertèbres, la face supérieure 6 étant destinée à venir au contact du plateau inférieur de la vertèbre sus-jacente tandis que la face inférieure 7 est destinée à venir au contact du plateau supérieur de la vertèbre sous-jacente. De la même façon, les termes "proximal(e)" et "distal(e)" utilisés dans la présente description sont à considérer, de façon classique, par rapport au praticien, "proximale" qualifiant une partie de la cage 1 (ou des autres implants décrits) se trouvant plus proche de ce praticien au cours de la mise en place de l'implant, et "distale" qualifiant donc une partie de la cage se trouvant plus éloignée de ce praticien au cours de cette même mise en place.

Il apparaît que le corps de cage 2 présente la forme d'un U horizontal, c'est-à-dire qu'il présente une branche supérieure 10, une branche inférieure 11 en vis-à-vis de la branche 10 et une portion intermédiaire 12 reliant ces deux branches l'une à l'autre.

Les deux branches 10, 11 forment des nervures antérieures 13 et sont séparées par une fente longitudinale 15 s'étendant depuis l'extrémité proximale 8 en direction d'une extrémité distale 9, cette fente 15 s'interrompant, du côté de cette extrémité distale 9, au niveau d'une paroi interne courbe que forme ladite portion intermédiaire 12. La fente 15 s'étend dans un plan parallèle à celui selon lequel les branches 10 et 11 s'étendent parallèlement l'une à l'autre et débouche dans les faces latérales de la cage 1, c'est-à-dire dans les faces de celle-ci qui sont perpendiculaires aux faces supérieure 6 et inférieure 7. Dans l'exemple de réalisation représenté, la fente 15 a une épaisseur, c'est-à-dire une dimension perpendiculaire audit plan, qui va en augmentant depuis l'extrémité proximale 8 vers l'extrémité distale 9 de la cage 1.

En référence à la figure 3, il apparaît que les faces internes des branches 10 et 11, c'est-à-dire les faces en vis-à-vis l'une de l'autre, sont inclinés de manière à converger l'une vers l'autre de l'extrémité proximale 8 de la cage 1 vers l'extrémité distale 9 de celle-ci ; ces faces forment des rampes le long desquelles le coin 3 est, après rupture des ponts 4, apte à se déplacer, comme cela sera décrit plus loin, ce déplacement permettant un écartement des deux branches 10, 11 l'une de l'autre.

Il apparaît également sur cette figure 3 que les nervures antérieures 13 sont transversales à la direction longitudinale de la cage 1 et qu'elles permettent de refermer partiellement, au niveau antérieur de cette cage, l'espace délimité intérieurement par le corps de cage 2.

Le coin 3 présente une section transversale carrée et forme, du côté postérieur de ce coin 3, des faces supérieure et inférieure venant à proximité immédiate des faces internes des branches 10 et 11. Ces faces supérieure et inférieure sont aptes à prendre appui contre ces faces internes au cours du déplacement du coin 3, pour réaliser l'écartement antérieur des branches 10 et 11. Du côté antérieur, le coin 3 présente des portions renfoncées ayant un contour qui suit celui des nervures 13. Ces dernières forment ainsi des portions d'enveloppement partiel du coin 3, assurant, après rupture des ponts 4, la rétention de ce coin 3 dans l'espace que délimite intérieurement le corps de cage 2.

Il apparaît sur la figure 2 que les quatre ponts 4 sont situés à proximité des angles que forme le coin 3, et il apparaît sur cette figure 2 et sur la figure 3 que ces ponts 4 des sections réduites, inférieures à 1 mm² pour chaque pont 4, de telle sorte que ces ponts 4 sont dimensionnés pour être ruptibles.

Le corps de cage 2 présente en outre extérieurement, au niveau de ses parois latérales, des zones renfoncées antérieures 16 qui permettent sa saisie et son maintien au moyen d'un instrument de mise en place de la cage 1 dans un espace intervertébral. Cet instrument (non représenté) est sous la forme d'une pince dont les deux branches forme une mâchoire de saisie de la cage 1 au niveau des zones renfoncées 16. L'instrument comprend également une tige mobile longitudinalement, entre les mors formant ladite mâchoire.

Les ponts 4 peuvent être rompus en usine, en fin de fabrication de la cage 1, auquel cas ladite tige réalise uniquement le déplacement du coin 3 ; ces ponts 4 pourraient être rompus une fois la cage 1 mise en place, auquel cas ladite tige réalise la rupture des ponts 4 puis le déplacement du coin 3.

Ce déplacement du coin 3 par rapport au corps de cage 2 dans la direction distale réalise un écartement des portions proximales d'extrémité des branches 10 et 11, de sorte que la cage 1 est apte de redonner à des vertèbres lombaires entre lesquelles elle est implantée leur écartement mutuel et leur lordose anatomiques.

La cage 1 représenté sur les figures 1 à 3 présente une largeur limitée, adaptée à une mise en place de cette cage sur un côté latéral d'un espace intervertébral ; une autre cage 1, identique, est dans ce cas mise en place sur l'autre côté latéral dudit espace intervertébral.

La cage 21 représentée sur la figure 4 présente une structure similaire à celle de la cage 1, sinon qu'elle est beaucoup plus large et adaptée à une mise en place centrale dans un espace intervertébral.

Cette cage 21 comprend un corps de cage 22, un coin d'écartement 23 et des ponts 24 de liaison de ce coin à ce corps de cage, la cage 21 étant réalisée par un procédé identique à celui précité utilisé pour réaliser la cage 1.

Dans ce cas, le coin 23 présente une forme en U, c'est-à-dire présente deux branches latérales engagées entre les portions latérales des branches supérieure et inférieure formées par le corps de cage 22, et présente une portion centrale reliée au corps de cage 22 par les ponts 24.

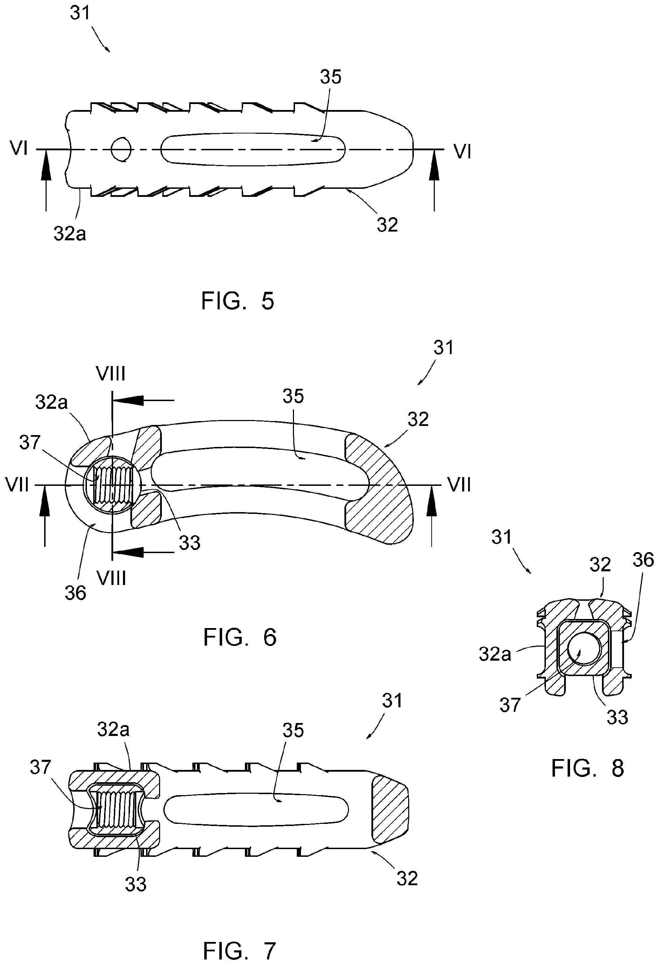

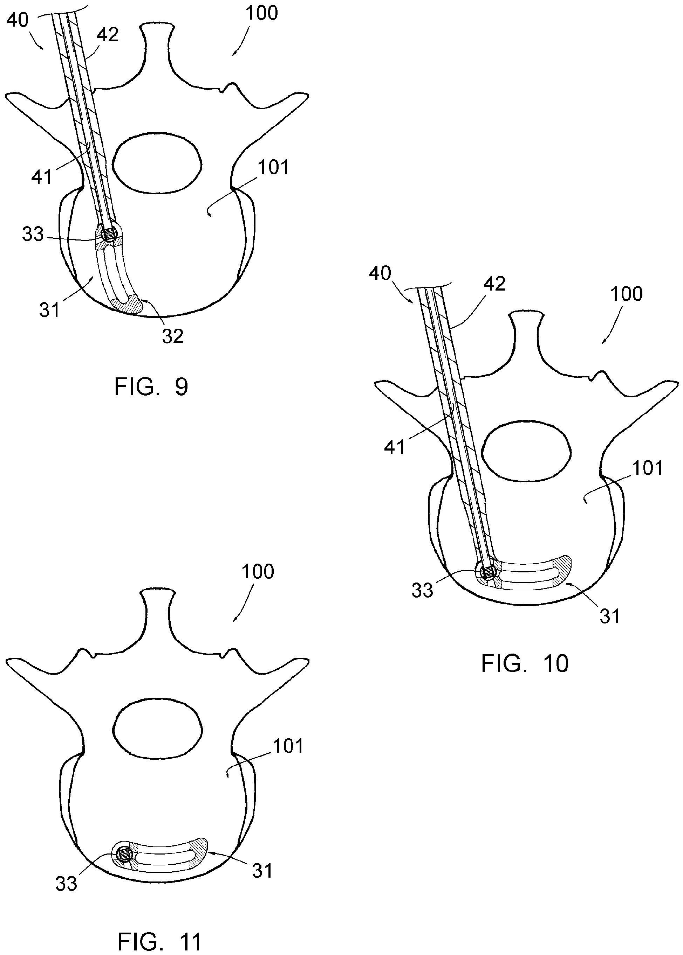

Les figures 5 à 8 représentent une cage intervertébrale 31 de type TLIF, c'est-à-dire destinée à être implantée par une voie d'abord transforaminale, comme cela apparaît sur les figures 9 à 11, au moyen d'un instrument 40.

La cage 31 comprend un corps de cage 32 et un cylindre 33 placé à l'intérieur d'un palier que forme une portion proximale 32a du corps de cage 32, cette portion proximale 32a enveloppant partiellement ce cylindre 33 de façon à assembler ce ce cylindre au corps de cage 32. La cage 31 est réalisée par le même procédé que celui utilisé pour fabriquer les cages 1 et 21, et le cylindre 33 est relié au corps de cage 32 par un ou plusieurs ponts de matière, non visibles à l'échelle des figures.

Le corps de cage 32 présente une forme générale courbe, adaptée à une mise en place sur le côté antérieur d'un espace intervertébral 101, comme visible sur les figures 9 à 11, et forme une cavité 35 de réception de greffons ou de copeaux d'os.

Il apparaît sur les figures 6 et 8 le corps de cage 32 forme, au niveau de la portion 32a, une lumière courbe 36 dont la courbure est centrée sur l'axe dudit palier, cette lumière s'étendant sur environ 90°, depuis une extrémité située légèrement en retrait vis-à-vis de l'axe longitudinal du corps de cage 32 jusqu'à son autre extrémité, située sur le côté latéral concave du corps 32.

Le cylindre 33 est engagé de manière ajustée dans ledit palier mais, après rupture du ou des ponts le reliant au corps de cage 32, est apte à pivoté dans ce palier autour d'un axe perpendiculaire aux faces supérieure et inférieure du corps de cage 32. Ce cylindre 33 est percé d'un trou 37 formant un filet intérieurement, qui le traverse entièrement, dans lequel est apte à être vissé et dévissé l'embout fileté d'une tige 41 que comprend l'instrument 40.

Il apparaît sur les figures 9 à 11 que l'instrument 40, outre cette tige 41, comprend un corps 42 apte à venir en appui contre ladite portion proximale 32a. Ce corps 42 est traversé par la tige 41, laquelle est mobile axialement et en rotation à l'intérieur de lui.

Les ponts précités sont rompus en usine, au moment de la fabrication de la cage 31.

Comme cela se comprend en référence aux figures 6 à 9, la tige 41 est vissée au travers du trou 37 jusqu'à prendre appui contre le corps de cage 32, avec ce dernier placé dans une position angulaire telle que le trou 37 soit orienté parallèlement à l'axe longitudinal de ce corps de cage 32 (cet axe longitudinal étant celui selon lequel est opérée la coupe selon la ligne VII-VII), la cage 31 étant ainsi alignée avec l'instrument 40. Cet instrument 40 peut donc être utilisé pour l'insertion de la cage 31 dans un espace intervertébral 101 situé entre deux vertèbres 100, jusqu'à ce que la cage 31 vienne en appui contre les ligaments antérieurs (non représentés) reliant ces vertèbres 100, comme visible sur la figure 9. Une fois l'insertion de la cage 31 faite, la tige 41 est dévissée de façon à permettre le pivotement de cette cage par rapport à l'axe longitudinal de l'instrument 40, jusqu'à la adéquate pour le positionnement de la cage 31 dans l'espace intervertébral 101, comme visible sur la figure 10.

La tige 41 peut alors être dévissée de façon à être extraite du trou 37, permettant le retrait de l'instrument 40, comme montré sur la figure 11, ce dévissage assurant une séparation facile à réaliser de l'instrument 40 et de la cage 31.

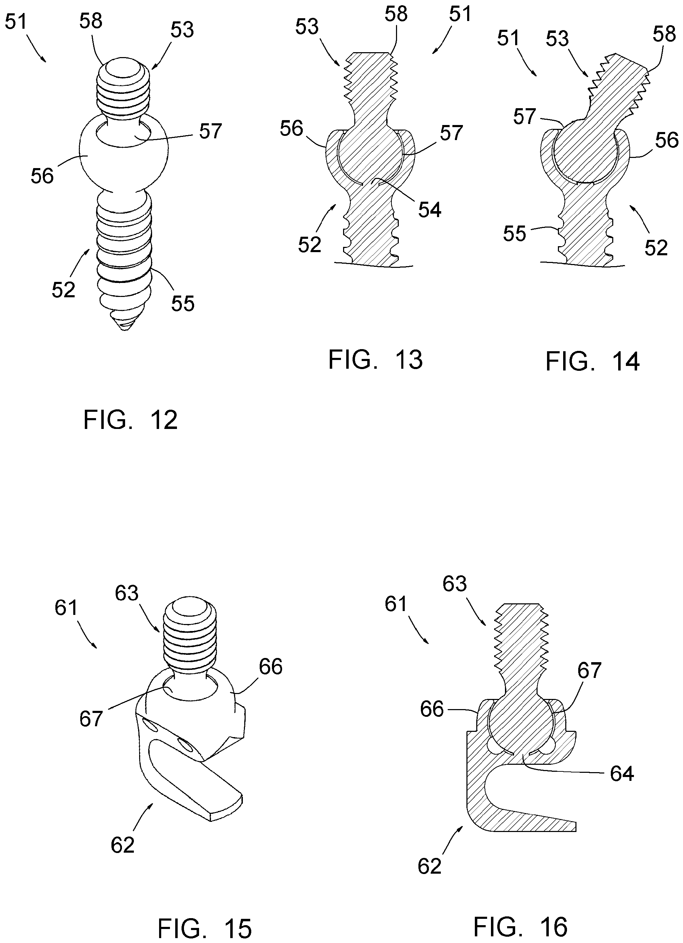

Les figures 12 à 14 représentent une vis pédiculaire 51 notamment utilisable pour la fixation d'un matériel d'ostéosynthèse vertébrale à au moins deux vertèbres devant être immobilisées les unes par rapport aux autres, selon une technique bien connue, notamment par la publication de demande de brevet précitée N° WO 98/55038.

Cette vis pédiculaire 51 comprend un corps 52, un pion proximal 53 destiné à être articulé par rapport à ce corps 52 et un pont ruptible 54 réalisant une liaison de ce pion proximal 53 à ce corps 52. L'ensemble est réalisé par le même procédé d'impression 3D que celui utilisé pour fabriquer les cages précitées.

Le corps 52 forme un filet distal 55 d'ancrage au pédicule d'une vertèbre et une tête 56 délimitant intérieurement une cavité de forme sphérique dans laquelle est contenue une tête sphérique 57 du pion 53. La paroi formant cette tête 56 est refermée sur cette tête 57, de sorte que le pion 53 reste assemblé au corps 52 lorsque le pont 54 est rompu.

Le pion 53, outre cette tête 57, présente un corps de pion fileté 58, destiné à recevoir une pièce de connexion à une barre vertébrale et un écrou de serrage de cette pièce de connexion entre lui et la tête 56, selon une technique également bien connue.

Sur les figures 15 et 16 est représenté un crochet lamaire 61 très similaire à la vis 51, comprenant donc un corps 62, un pion proximal fileté 63 et un pont ruptible 54 de liaison libérable de ce pion vis-à-vis de ce corps, l'ensemble étant aussi fabriqué par le même procédé que celui précité. Le corps de crochet 62 forme une tête 66 délimitant une cavité sphérique de réception d'une tête sphérique 67 correspondante, à l'identique de ce qui a été décrit précédemment s'agissant de la vis 51.

Il apparaît de ce qui précède que l'invention fournit un procédé de fabrication d'un implant, notamment vertébral ou intervertébral, présentant les avantages déterminants de permettre de réaliser un implant de façon relativement simple et rapide, sans opérations d'assemblage ultérieures.

L'invention a été décrite ci-dessus en référence à des formes de réalisation données uniquement à titre d'exemples. Il va de soi qu'elle s'étend à toutes les autres formes de réalisations couvertes par les revendications ci-annexées.

## Revendications

1. Procédé de fabrication d'un implant (1, 21, 31, 51, 61), notamment vertébral ou intervertébral, cet implant (1, 21, 31, 51, 61) comprenant au moins deux pièces assemblées, dont au moins une première pièce (3, 23, 33, 53, 63) est destinée à être mobile par rapport à au moins une deuxième pièce (2, 22, 32, 52, 62), le procédé comprenant l'étape consistant à concevoir l'implant (1, 21, 31, 51, 61) de telle sorte que ladite deuxième pièce (2, 22, 32, 52, 62) enveloppe au moins partiellement ladite première pièce (3, 23, 33, 53, 63) ;
**caractérisé en ce qu'**il comprend en outre les étapes suivantes :
- concevoir l'implant de telle sorte qu'au moins un pont de liaison (4, 24, 54, 64) relie ladite première pièce à ladite deuxième pièce, chaque pont présentant une section telle qu'il est ruptible ;
- réaliser l'implant (1, 21, 31, 51, 61) par un procédé dit d"'impression 3D", consistant :
- à déposer sur un plateau une couche de poudre de matériau apte à être fusionné,
- à opérer une fusion des particules de cette couche de poudre en des emplacements correspondant à la forme qu'ont, au niveau de cette couche, ladite première pièce (3, 23, 33, 53, 63) et/ou ladite deuxième pièce (2, 22, 32, 52, 62) et/ou ledit au moins un pont (4, 24, 54, 64), selon que cette première pièce, cette deuxième pièce et/ou ce pont sont présents ou non au niveau de la section qu'a l'implant à constituer au niveau de cette couche ;
- à reproduire ces opérations jusqu'à la constitution complète de l'implant (1, 21, 31, 51, 61), et
- à éliminer les particules de poudre non fusionnées.

2. Implant (1) obtenu par le procédé selon la revendication 1, **caractérisé en ce que** cet implant est une cage intervertébrale (1, 21) ayant une partie antérieure dont la hauteur est apte à être augmentée après implantation ; ladite deuxième pièce est constituée par un corps de cage (2, 22) formant deux branches (10, 11), qui est déformable de cette sorte que les portions d'extrémité antérieures de ces branches (10, 11) soient mobiles l'une par rapport à l'autre entre une position de rapprochement mutuel et une position d'écartement mutuel ; ladite première pièce est constituée par un coin d'écartement (3) engagé entre lesdites branches (10, 11), relié au corps de cage (2, 22) par ledit au moins un pont (4).

3. Implant (31) obtenu par le procédé selon la revendication 1, **caractérisé en ce que** l'implant est une cage intervertébrale (31) dans laquelle ladite première pièce est constituée par une pièce de révolution (33) et ladite deuxième pièce est constituée par un corps de cage (32) formant un palier de réception et rétention de cette pièce de révolution (33) ; la pièce de révolution (33) comprend un moyen (37) de liaison à un instrument (40) d'introduction / positionnement de l'implant (31).

4. Implant (31) selon la revendication 3, **caractérisé en ce que** ladite pièce de révolution est un cylindre (33).

5. Implant (51) obtenu par le procédé selon la revendication 1, **caractérisé en ce que** cet implant est une vis pédiculaire polyaxiale (51) ou un crochet lamaire polyaxial (61) ; ladite première pièce est formée par une tête de connexion ou un pion fileté (53, 63), et ladite deuxième pièce (52, 62) est destinée à venir en engagement avec une vertèbre à traiter.

6. Implant (51, 61) selon la revendication 5, **caractérisé en ce que** l'une parmi ladite première pièce (53, 63) et ladite deuxième pièce (52, 62) présente une portion de forme au moins partiellement sphérique et l'autre parmi cette première pièce (53, 63) et cette deuxième pièce (52, 62) présente une cavité de forme au moins partiellement sphérique destinée à recevoir cette portion de forme au moins partiellement sphérique, ledit pont de matière (54, 64) étant aménagé entre cette portion de forme au moins partiellement sphérique et la paroi délimitant cette cavité de forme au moins partiellement sphérique.

## Patentansprüche

1. Verfahren zum Herstellen eines Implantats (1, 21, 31, 51, 61), insbesondere eines Wirbel- oder Zwischenwirbel-Implantats, wobei dieses Implantat (1, 21, 31, 51, 61), wenigstens zwei zusammengefügte Teile umfasst, von denen wenigstens ein erstes Teil (3, 23, 33, 53, 63) dazu bestimmt ist, relativ zu wenigstens einem zweiten Teil (2, 22, 32, 52, 62) beweglich zu sein, wobei das Verfahren den Schritt umfasst, darin bestehend, das Implantat (1, 21, 31, 51, 61) so zu auszubilden, dass das zweite Teil (2, 22, 32, 52, 62) wenigstens teilweise das erste Teil (3, 23, 33, 53, 63) umhüllt;
**dadurch gekennzeichnet, dass** es ferner die folgenden Schritte umfasst:
- Ausbilden des Implantats in der Form, dass wenigstens eine Materialbrücke (4, 24, 54, 64) das erste Teil mit dem zweiten Teil verbindet, wobei jede Brücke einen derartigen Abschnitt aufweist, dass er brechbar ist;
- Realisieren des Implantats (1, 21, 31, 51, 61) durch ein 3D-Druckverfahren, bestehend im:
- Abscheiden auf einer Platte einer Pulverschicht aus einem schmelzbaren Werkstoff,
- Durchführen einer Schmelzung der Partikel dieser Pulverschicht an Stellen der Form, die, im Bereich dieser Schicht, das erste Teil (3, 23, 33, 53, 63) und/oder das zweite Teil (2, 22, 32, 52, 62) und/oder die wenigstens eine Brücke (4, 24, 54, 64) aufweisen, je nachdem, ob dieses erste Teil, dieses zweite Teil und/oder diese Brücke im Bereich des Abschnitts, den das zu bildende Implantat im Bereich dieser Schicht aufweist, vorhanden sind oder nicht;
- Wiederholen dieser Arbeitsgänge bis zur vollständigen Bildung des Implantats (1, 21, 31, 51, 61) und
- Entfernen der nicht geschmolzenen Pulverpartikel.

2. Implantat (1), erhalten durch das Verfahren nach Anspruch 1, **dadurch gekennzeichnet, dass** dieses Implantat ein Zwischenwirbel-Käfig (1, 21) ist mit einem vorderen Teil, dessen Höhe nach Implantation vergrößerbar ist; das zweite Teil von einem Käfigkörper (2, 22) gebildet ist, der zwei Schenkel (10, 11) bildet, der so verformbar ist, dass die vorderen Endteile dieser Schenkel (10, 11) zueinander beweglich sind zwischen einer zueinander herangeführten Position und einer voneinander abgespreizten Position; das erste Teil von einem Abspreizkeil (3) gebildet ist, der zwischen den zwei Schenkeln (10, 11) eingesetzt ist und mit dem Käfigkörper (2, 22) durch die wenigstens eine Brücke (4) verbunden ist.

3. Implantat (31), erhalten durch das Verfahren nach Anspruch 1, **dadurch gekennzeichnet, dass** das Implantat ein Zwischenwirbel-Käfig (3) ist, wobei das erste Teil von einem Rotationsteil (33) gebildet ist und das zweite Teil von einem Käfigkörper (32) gebildet ist, der ein Lager zum Aufnehmen und Rückhalten dieses Rotationsteils (33) bildet; das Rotationsteil (33) ein Mittel (37) zum Verbinden mit einem Instrument (40) zum Einführen/Positionieren des Implantats (31) umfasst.

4. Implantat (31) nach Anspruch 3, **dadurch gekennzeichnet, dass** das Rotationsteil ein Zylinder (33) ist.

5. Implantat (51), erhalten durch das Verfahren nach Anspruch 1, **dadurch gekennzeichnet, dass** dieses Implantat eine polyaxiale Pedikelschraube (51) oder ein polyaxialer Laminahaken (61) ist; das erste Teil von einem Verbindungskopf oder einem Gewindestift (53, 63) gebildet ist und das zweite Teil (52, 62) dazu dient, mit einem zu behandelnden Wirbel in Eingriff zu kommen.

6. Implantat (51, 61) nach Anspruch 5, **dadurch gekennzeichnet, dass** eines von dem ersten Teil (53, 63) und dem zweiten Teil (52, 62) einen Teil mit wenigstens teilweise sphärischer Form aufweist und das andere von diesem ersten Teil (53, 63) und diesem zweiten Teil (52, 62) eine Vertiefung mit wenigstens teilweise sphärischer Form aufweist, die dazu bestimmt ist, diesen Teil mit wenigstens teilweise sphärischer Form aufzunehmen, wobei die Materialbrücke (54, 64) zwischen diesem Teil mit wenigstens teilweise sphärischer Form und der Wand, die diese Vertiefung mit wenigstens teilweise sphärischer Form begrenzt, angeordnet ist.

## Claims

1. Method for manufacturing an implant (1, 21, 31, 51, 61), in particular vertebral or intervertebral, this implant (1, 21, 31, 51, 61) comprising at least two components, at least a first component (3, 23, 33, 53, 63) of which is intended to be movable relative to at least a second component (2, 22, 32, 52, 62), the method comprising the step consisting of designing the implant (1, 21, 31, 51, 61) such that said second component (2, 22, 32, 52, 62) at least partially envelops said first component (3, 23, 33, 53, 63);
**characterized in that** it further includes the following steps:
- designing the implant such that at least one connecting bridge (4, 24, 54, 64) connects the first component to said second component, each bridge having a section such that it is frangible;
- making the implant (1, 21, 31, 51, 61) using a so-called "3D printing" method, consisting of:
- depositing on a platen, a layer of powdered fusible material,
- causing the particles of this layer of powder to fuse in locations corresponding to the shape which, at this layer, said first component (3, 23, 33, 53, 63) and/or said second component (2, 22, 32, 52, 62) and/or said at least one bridge (4, 24, 54, 64) have, depending on whether said first component, said second component and/or said bridge are present at the section the implant to be formed has at said layer;
- repeating these operations until the implant (1, 21, 31, 51, 61) is fully constructed, and
- eliminating the powder particles that are not fused.

2. Implant (1) obtained by the method according to claim 1, **characterized in that** this implant is an intervertebral cage (1, 21) having an anterior part, the height of which is able to be increased after implantation; said second component is then made up of a cage body (2, 22) forming two branches (10, 11), which is deformable in this way such that anterior end portions of these branches (10, 11) are movable relative to one another between a close together position and a separated position; said first component is then made up of a separating wedge (3) engaged between said branches (10, 11), connected to the cage body (2, 22) by said at least one bridge (4).

3. Implant (31) obtained by the method according to claim 1, **characterized in that** the implant is an intervertebral cage (31) wherein said first component is made up of a component of revolution (33) and said second component is made up of a cage body (32) forming a bearing for receiving and retaining this component of revolution (33); the component of revolution (33) includes a means (37) for connecting to an instrument (40) for inserting / positioning the implant (31).

4. Implant (31) according to claim 3, **characterized in that** said component of revolution is a cylinder (33).

5. Implant (51) obtained by the process according to claim 1, **characterized in that** this implant is a polyaxial pedicle screw (51) or a polyaxial laminar hook (61); said first component is formed by a connecting head or a threaded pin (53, 63), and said second component (52, 62) is intended to be engaged with a vertebra to be treated.

6. Implant (51, 61) according to claim 5, **characterized in that** one from among said first component (53, 63) and said second component (52, 62) has an at least partially spherical portion and the other from among the first component (53, 63) and the second component (52, 62) has a corresponding at least partially spherical cavity intended to receive said at least partially spherical portion, said bridge (54, 64) being arranged between said at least partially spherical portion and the wall defining said at least partially spherical cavity.
